# EUROPÄISCHE PATENTANMELDUNG

(11) **EP 1 050 293 A1**
(43) Veröffentlichungstag der Anmeldung: **08.11.2000**
(21) Anmeldenummer: 99810396.4
(22) Anmeldetag: 06.05.1999
(51) Int. Cl.: A61K 7/16

(54) **Zahnpflegemittel**

(71) Anmelder: HAWE NEOS DENTAL Dr. H. V. WEISSENFLUH AG, 6934 Bioggio (CH)
(72) Erfinder: Kilcher, Beat, 6935 Bosco Luganese (CH); Balmelli, Patrizia, 6932 Breganzona (CH); Silber, Gert, Dr., 6946 Ponte Capriasca (CH); von Weissenfluh, Beat A., 6925 Gentilino (CH)
(74) Vertreter: AMMANN PATENTANWAELTE AG BERN

(57) **Zusammenfassung**

Ein Zahnpflegemittel für die vorbeugende Zahnhygiene (Prophylaxepaste) weist als Hauptkomponenten auf: einen Putzkörper aus plättchenförmigen Gesteinspartikeln, bevorzugt Perlit in einem Anteil von 10 - 80 Gew.-%, bevorzugt 35 - 55 Gew.-%, ein nichtionisches, bevorzugt polymeres Tensid in einem Anteil von 20 - 80 Gew.-%, bevorzugt 40 - 50 Gew.-%, sowie 0 - 20 Gew.-%, bevorzugt 1 - 10 Gew.-% Emulgator bzw. -gemisch. Als Tensid ist Polyethylenglykol (Macrogol) mit Molekulargewichten von 200 - 1000, bevorzugt 200 - 600, in reiner Form oder als Gemisch verschiedener Varianten unterschiedlicher Molekulargewichte besonders geeignet. Das Zahnpflegemittel zeichnet sich durch geschmeidige Konsistenz und geringe Neigung zum Verspritzen aus.

## Beschreibung

Die vorliegende Erfindung betrifft ein Zahnpflegemittel zur vorbeugenden Zahnhygiene gemäss Anspruch 1.

Ein derartiges Zahnpflegemittel besteht in der Regel aus drei verschiedenen, nach Härte eingeordneten Pasten (hart, mittel, weich), deren Struktur nicht mit einer üblicherweise allgemein verwendeten Zahnpasta vergleichbar ist. Sie entspricht eher einer fast krümeligen Polierpaste. Diese krümelige Struktur ist bei Anwendung in der Mundhöhle mit einem Poliergerät notwendig, um Substanzverlust durch z.B. Spritzen zu vermeiden. Die Pasten werden in Ergänzung zur täglichen Zahnpflege von Zahnhygienikern benutzt, um hartnäckig anhaftende Zahnbeläge zu entfernen. Diese zusätzliche Behandlung soll die Zahnpflege derart unterstützen, dass Karies verringert und somit zahnärztliche Behandlung weitgehend überflüssig wird.

Eine derartige wasserhaltige Paste, die Fällungskieselsäure als hauptsächliche Putzkörperkomponente, daneben aber auch Perlit enthält, ist in der EP-A-0 268 763 beschrieben. Eine andere Variante, die zwingend hydrophobe pyrogene Kieselsäure zur Stabilisierung des Perlits enthält, ist in EP-A-528 756 beschrieben.

In der Anwendung zeigen diese Zahnpflegemittel, auch Prophylaxepasten genannt, verschiedene Probleme:

Die zum Zerbröseln neigende Konsistenz erschwert das Entnehmen aus Vorrats- oder Portionenbehältern und das Aufbringen auf die Zahnreinigungswerkzeuge (Pastenträger) der Zahnhygieniker.

Aus dem gleichen Grund ist es auch nicht möglich, die bekannten Prophylaxepasten in die bevorzugten Portionen für jeweils eine Anwendung problemlos zu portionieren.

Schliesslich zeigen die bekannten Prophylaxepasten beim Kontakt mit der Saliva des Patienten die bekannte Neigung, sich spontan mit dieser zu vermischen und vom Pastenträger heruntergeschleudert zu werden. Diese Werkzeuge zeichnen sich z.B. durch einen Drehzahlbereich von 2000 bis 6000 Umdrehungen pro Minute aus. Es wird jedoch oft empfohlen, keine Drehzahlen oberhalb 3000 Umdrehungen pro Minute einzusetzen, da ansonsten die Paste vom Werkzeug heruntergeschleudert wird.

Eine Aufgabe der vorliegenden Erfindung besteht daher darin, ein Zahnpflegemittel für die vorbeugende Zahnhygiene anzugeben, das verbesserte Anwendungseigenschaften aufweist.

Ein derartiges Zahnpflegemittel ist im Anspruch 1 angegeben. Die weiteren Ansprüche geben bevorzugte Ausführungsformen und Anwendungen an.

Die erfindungsgemässe Paste zeichnet sich demgemäss dadurch aus, dass sie einen Putzkörper, der im wesentlichen aus scharfkantigen, plättchenförmigen Partikeln eines Gesteins, bevorzugt Perlit, besteht, und ein nichtionisches Tensid enthält.

Das nichtionische Tensid übernimmt zugleich die Funktion eines Lösungsmittels, sein Anteil und seine Art stellen also die Fliesseigenschaften des Zahnpflegemittels ein. Als besonders geeignet hat sich hierfür der unter dem INN Macrogol bekannte Polyethylenglykol, ein Polyether, erwiesen. Es wird vermutet, dass das nichtionische Tensid eine Art Hülle wenigstens um die Gesteinspartikel bildet, wobei diese Hülle zugleich als Bindungsvermittler zu den übrigen, freien Molekülen des Tensides wirkt. Aus dieser Überlegung heraus ist es verständlich, dass insbesondere mässig langkettige Tenside besonders gute Wirksamkeit aufweisen. Versuche haben aber auch gezeigt, dass zu langkettige Spezies dazu führen, dass die Paste ihre Geschmeidigkeit verliert und zu trocken oder zu bröselig wird. Es hat sich auch gezeigt, dass in der Regel ein Zusatz eines Emulgators oder eines Emulgatorgemischs erforderlich ist, insbesondere wenn auch bei sorgfältiger Auswahl des Tensids keine genügende Geschmeidigkeit erreicht wird.

Plättchenförmige Putzkörperpartikel neigen dazu, sich in der Anwendung derart auszurichten, dass Riefenbildung und andere unerwünschte Folgen der Einwirkung der Prophylaxepaste gegenüber z.B. eher kugeligen, scharfkantigen Partikeln deutlich verringert werden. Aus diesem Befund heraus kann erwartet werden, dass auch andere mineralische Materialien, also Gesteine, die zu Partikeln geeigneter Grösse und Form zerkleinerbar sind, ähnlich dem Prototypen Perlit verwendbar sind. Die Plättchen können dabei auch gekrümmt sein, so wie diejenigen von Perlit schalenförmig sind ähnlich Eierschalenfragmenten. Bemerkenswert ist auch hinsichtlich Perlit, dass die Partikel bei der Anwendung zerbrechen, dabei aber scharfkantig bleiben und damit ihre Reinigungswirkung bewahren, wenn auch zunehmend schonender werden entsprechend einem Übergehen von grobem zu feinerem Schleifmaterial. Weitere Details der Verwendung von Perlit und anderen Gesteinsarten in Prophylaxepasten sind der EP-A-528 756 zu entnehmen.

Die Erfindung soll weiter an einem Ausführungsbeispiel erläutert werden. Prozentangaben sind, wie überall in der Beschreibung und den Ansprüchen, Gewichtsprozente.

Eine erfindungsgemässe Prophylaxepaste weist folgende obligatorischen Komponenten auf:
- Perlit (expandiert) 10 - 80 Gew.-%,
   bevorzugt 35 - 55 Gew.-%
- Macrogol 20 - 80 Gew.-%,
   bevorzugt 40 - 50 Gew.-%
- Emulgator oder -gemisch 0 - 20 Gew.-%,
   bevorzugt 1 - 10 Gew.-%

Das Macrogol ist dabei jeweils ein Produkt mit bestimmtem Molekulargewicht, wie sie käuflich sind. Es übernimmt als eine Hauptkomponente die Funktionen der üblicherweise vorhandenen Flüssigkeit wie Wasser, und sein Anteil und seine Art beeinflussen die Rheologie, insbesondere Viskosität und Geschmeidigkeit. Die Paste ist mithin im wesentlichen frei von Wasser und anderen niedermolekularen, bei Zimmertemperatur (298 K) verwendbaren Lösungsmitteln wie den in dieser Anwendung bekannten Stoffen Ethanol, Propylenglykol, Glycerin. Als niedermolekular könnte also ein Molekulargewicht von höchstens 100 angesehen werden.

Molekulargewichte von Macrogol von 200 - 1000, bevorzugt 200 - 600, haben sich als besonders geeignet erwiesen. Es können die marktgängigen Produkte mit eng spezifiziertem, mittlerem oder eindeutigem Molekulargewicht pur oder in Mischung verwendet werden. Es ist zu erwarten, dass durch Mischen von Tensiden verschiedenen Molekulargewichts eine feinere Einstellung der Anwendungseigenschaften, insbesondere der Rheologie, möglich ist. Bei einem zunehmenden Anteil an höhermolekulargewichtigem Macrogol ist z.B. eine Erhöhung der Viskosität zu erwarten.

Das Perlit weist einen mittleren Partikeldurchmesser von ca. 30 µm auf, wobei sich 99 % im Grössenbereich von 1 µm bis 200 µm Durchmesser befinden.

Als Emulgator bzw. Komponenten des Emulgatorgemisches können dienen:
Höhere Fettalkohole, insbesondere mit 8 bis 20
Kohlenstoffatomen in der Kette, z.B. Cetylalkohol, Laurylalkohol, Stearylalkohol und/oder
Fettsäureester eines Polyoxyethylens, z.B. ethoxyliertes Rizinusöl (Emulgin RO40, Henkel KG a.A., Deutschland) oder Polyoxyethylenstearat.

Zusätzlich kann die Prophylaxepaste noch die üblichen Hilfs- und Wirkstoffe in den nötigen Anteilen aufweisen:

Antiseptikum, Fluoridsalz, Aromen, Süssstoff, Konservierungsmittel (Antioxidanz, Antimikrobiotikum) und evtl. zusätzliche, sekundäre Abrasivmaterialien. Die genannten sekundären Abrasivkörper stellen zusammen mit dem Perlit, oder allgemein Gestein, den Putzkörper dar.

In der Praxis wurde gefunden, dass die so herstellbare Prophylaxepaste sehr gut in die gewünschten Einmal-Dosen portionierbar ist, insgesamt eine eher cremige Konsistenz im Vergleich mit den bekannten Pasten aufweist und trotzdem weniger dazu neigt, bei Kontakt mit Saliva vom Werkzeug heruntergeschleudert zu werden. Bemerkenswert ist, dass die Paste gegenüber Wasseraufnahme tolerant ist, d.h. ihre Anwendungseigenschaften in feuchter Umgebung nur so wenig ändert bzw. sie hinreichend lange bewahrt, dass sie wirksam bleibt und insbesondere das Verspritzen in wesentlich geringerem Ausmass als bei bekannten Pasten auftritt.

Ein weiterer Vorteil ist die gefühlsmässig angenehme, cremige Konsistenz.

Ausgehend von der Beschreibung des Ausführungsbeispieles sind dem Fachmann Varianten im Bereich der Erfindung zugänglich. Denkbar ist es, modifizierte Polyethylenglykole einzusetzen, z.B. mit entsprechenden Substituenten an der Molekülkette oder den Enden, verzweigte Arten, veresterte oder veretherte Formen, usw. Denkbar sind auch andere Strukturelemente der Polymerkette wie z.B. Propylen oder Methylen sowohl als Homopolymer wie auch als Copolymer. Bei der Wahl des Gesteins und dessen Partikelgrössen ist ein grosser Spielraum für die Anpassung an den Einsatzzweck vorhersehbar.

Denkbar ist auch der Zusatz von niedermolekularen Lösungsmitteln (Wasser, Propylenglykol etc.) in geringen Mengen, bevorzugt in einem Gesamtanteil von höchstens 10 Gew.-%, weiter bevorzugt höchstens 5 Gew.-%, um eine Optimierung der Konsistenz durchzuführen.

## Patentansprüche

1. Zahnpflegemittel für die vorbeugende Zahnhygiene, dadurch gekennzeichnet,
dass es plättchenförmige, ebene oder gekrümmte Partikel eines Gesteins, insbesondere von Perlit, als hauptsächlichen Bestandteil des Putzkörpers, eine effektive Menge eines nichtionischen Tensids und einen Emulgatoranteil aufweist, Putzkörper und Tensid zusammen mindestens 30 Gew.-%, bevorzugt mindestens 70 Gew.-% des Zahnpflegemittels darstellen und der Emulgatoranteil 0 - 20 Gew.-% beträgt.

2. Zahnpflegemittel gemäss Anspruch 1, dadurch gekennzeichnet, dass es höchstens 10 Gew.-%, bevorzugt höchstens 5 Gew.-% Wasser und andere niedermolekulare, insbesondere ein Molekulargewicht von höchstens 100 aufweisende, bei 298 K flüssige und als Lösungsmittel dienende Substanzen wie Alkohole enthält und insbesondere bevorzugt im wesentlichen frei von diesen Komponenten ist.

3. Zahnpflegemittel gemäss Anspruch 1 oder 2,
dadurch gekennzeichnet,
dass es 10 - 80 Gew.-%, bevorzugt 35 - 55 Gew.-% Perlit aufweist.

4. Zahnpflegemittel gemäss einem der Ansprüche 1 bis 3, dadurch gekennzeichnet, dass das Tensid ein Polyether, bevorzugt ein Polyalkylether ist.

5. Zahnpflegemittel gemäss Anspruch 4, dadurch gekennzeichnet, dass das Tensid ein modifizierter oder unmodifizierter Polyalkylenglykol ist, bevorzugt mit Alkylenglykolgruppen mit 1 bis 4 Kohlenstoffatomen und weiter bevorzugt mit im wesentlichen ausschliesslich Ethylenglykol-Gruppen.

6. Zahnpflegemittel gemäss einem der Ansprüche 1 bis 5, dadurch gekennzeichnet, dass es 20 - 80 Gew.-%, bevorzugt 40 - 50 Gew.-% Macrogol (Polyethylenglykol) aufweist.

7. Zahnpflegemittel gemäss einem der Ansprüche 1 bis 6, dadurch gekennzeichnet,
dass sich das Tensid aus Molekülen mit einem Molekulargewicht im wesentlichen im Bereich von 200 bis 1000, bevorzugt 200 bis 600, zusammensetzt.

8. Zahnpflegemittel gemäss einem der Ansprüche 1 bis 7, dadurch gekennzeichnet,
dass zusätzlich mindestens eine der folgenden Komponenten vorhanden ist:
- Antiseptikum
- Fluoridsalz
- Aromastoff
- Süssstoff
- Konservierungsmittel, insbesondere Antibiotikum, Antioxidanz
und/oder
- mindestens ein sekundärer Abrasivstoff im Putzkörper.

9. Zahnpflegemittel gemäss einem der Ansprüche 1 bis 8, dadurch gekennzeichnet,
dass der Emulgatoranteil 1 - 10 Gew.-% beträgt.

10. Zahnpflegemittel gemäss einem der Ansprüche 1 bis 9, dadurch gekennzeichnet,
dass der Emulgatoranteil eine oder mehrere folgender Substanzen bzw. Substanzklassen umfasst:
- Höherer Fettalkohol, insbesondere mit 8 bis 20 Kohlenstoffatomen in der Kohlenstoffkette, wie z.B. Cetylalkohol, Stearylalkohol, Laurylalkohol,
- Fettsäureester von Polyoxyalkylethern, wie z.B. ethoxyliertes Rizinusöl, Polyoxyethylenstearat.

11. Verwendung des Zahnpflegemittels gemäss einem der Ansprüche 1 bis 10 mit einem Schleif- oder Polierinstrument der professionellen Zahnprophylaxe.
